# EUROPÄISCHE PATENTANMELDUNG

(11) **EP 3 680 916 A1**
(43) Veröffentlichungstag der Anmeldung: **15.07.2020**
(21) Anmeldenummer: 20020007.9
(22) Anmeldetag: 07.01.2020
(51) Int. Cl.: G16H 40/67, G16H 80/00, A61B 5/00, H04L 29/08, H04N 7/15

(54) **AUDIO-VIDEOKONFERENZSYSTEM DER TELEMEDIZIN**

(30) Priorität: 09.01.2019 DE 102019000067
(71) Anmelder: Lipp, Thomas, 04299 Leipzig (DE)
(72) Erfinder: Lipp, Thomas, 04299 Leipzig (DE)
(74) Vertreter: Müller, Volkmar

(57) **Zusammenfassung**

Die Erfindung betrifft ein Audio-Videokonferenzsystem der Telemedizin, welches jeweils ein Videokonferenz-Endgerät an einer Basisstation (2), welche am Praxisstandort geordnet ist, und einer Außenstation (3), welche am Patientenstandort angeordnet ist, besitzt.

## Beschreibung

Die Erfindung betrifft ein Audio-Videokonferenzsystem der Telemedizin, welches jeweils ein Videokonferenz-Endgerät an einer Basisstation, welche am Praxisstandort geordnet ist, und einer Außenstation, welche am Patientenstandort angeordnet ist, besitzt.

Derzeit sind eine Reihe von Audio-Videokonferenzsystemen der Telemedizin im Einsatz, wobei die Vitaldaten eines Patienten am Patientenstandort durch eine Krankenschwester oder dgl. gemessenen werden und nachfolgend zum Praxisstandort zu einem Datenspeicher übertragen werden. Der Hausarzt ruft dort die gemessenen Vitaldaten nachfolgend vom Datenspeicher ab und begutachtet diese.

Großstädte, unter- bzw. schJechtversorgte Gebiete, meist abgelegen und ländlich, wie auch Pflegeheime und Einrichtungen des betreuten Wohnens, sind oft hausärztlich, aber noch mehr fachärztlich ungenügend versorgt mit der Folge von Unzufriedenheiten, vermehrten Rettungs-/Noteinsätzen, gehäuften Einweisungen und Krankentransporten, sprich schlechterer medizinischer und damit kostenintensiver Versorgung.

Derzeit werden beispielsweise Schwestern bzw. medizinisch geschultes Personal eingesetzt, welche zum Patienten außerhalb einer Arztpraxis fahren und dort am Standort des Patienten unter Verwendung üblicher medizinischer Messgeräte entsprechende Daten erheben und diese nachfolgend zur Arztpraxis in elektronischer Form übersenden.

Anschließend nimmt die Schwester vom Standort des Patienten aus Kontakt zum Arzt auf und stellt die Kommunikationsverbindung zwischen Arzt und Patient her.
Der Arzt hat keine Möglichkeit die Art und Weise der Erhebung dieser Daten aktiv zu beeinflussen bzw. zu überwachen. Außerdem liegen keine dieser Daten in Echtzeit vor, was eine deutliche Qualitätseinbuße darstellt. Außerdem ist es nicht möglich in Echtzeit weitere Fachkompetenz, beispielsweise eines Facharztes beizuziehen, um die Qualität der Diagnose zu verbessern bzw. zu beschleunigen.

Aufgabe der Erfindung ist es, ein technisch einfaches, sicheres und robustes Audio-Videokonferenzsystem mit einer Übertragungseinrichtung zur drahtlosen Übermittlung von Patientendaten sowie Patientenmessdaten bereit zu stellen, welches es insbesondere ermöglicht, eine Breitbandverbindung für eine Audio-Video-Verbindung zwischen zwei und mehr Standorten bereit zu stellen, welche es in Echtzeit ermöglicht, eine Audio-Videokonferenz sicher zu realisieren, um möglichst zeitnah zu einer qualitativ hochwertigen Diagnose zu gelangen. Dabei soll es ermöglicht sein, dass neben der eigentlichen Audio-Videokonferenz gleichzeitig auch die Patientenmessdaten dem Hausarzt am Praxisstandort und so erforderlich einem Facharzt an einem weiteren Standort in Echtzeit zur Verfügung stehen.

Die Aufgabe der Erfindung wird durch ein Audio-Videokonferenzsystem, welches die Merkmale des Anspruchs 1 besitzt, gelöst.

Erfindungswesentlich ist, dass das Audio-Videokonferenzsystem eine Übertragungseinrichtung 1 zur drahtlosen Übermittlung von Patientenmessdaten in Echtzeit von der Außenstation 3 an die Basisstation 2 und/oder Zusatzstation 4 besitzt, wobei die Außenstation 3 zumindest eine Datenschnittstelle zur Übernahme von Patientenmessdaten, welche durch Messungen von zumindest einem medizinischen Messgerät 8 am Patienten 9 ermittelt worden sind, besitzt und die Außenstation 3, welche zumindest ein medizinisches Messgerät 8 umfasst, tragbar ist.

Damit ist ermöglicht, dass die gemessenen Patientenmessdaten digital an die Außenstation in üblicher Art und Weise übergeben werden und in üblicher Art und Weise an die Basisstation übertragen werden können. Dies kann regelmäßig in bekannter Art und Weise verschlüsselter erfolgen. Die Patientenmessdaten stehen somit dem Hausarzt sowie erforderlich einem Facharzt während der Videokonferenz in sehr guter Qualität zur Verfügung.

Im Sinne der Erfindung befindet sich die Basisstation an dem Ort, insbesondere dem Praxisstandort des Hausarztes, an welchem sich der behandelnde Arzt/Hausarzt, nämlich die Bedienperson 5, zum Zeitpunkt der Videokonferenz befindet. Dabei kann die Zusatzstation auch eine mobile sein, beispielsweise ein Smartphone oder Tablett.

Im Sinne der Erfindung befindet sich die Zusatzstation an dem Ort, insbesondere einem Facharztstandort, an welchem sich der behandelnde Facharzt, nämlich die Bedienperson 7, zum Zeitpunkt der Videokonferenz befindet. Dabei kann die Zusatzstation auch eine mobile sein, beispielsweise ein Smartphone oder Tablett.

Im Sinne der Erfindung befindet sich die Außenstation an dem Ort, insbesondere am Patientenstandort, an welchem sich der zu behandelnde Patient und die Bedienperson 6 zum Zeitpunkt der Videokonferenz befinden.

Mit der erfindungsgemäßen Übertragungseinrichtung ist es in technisch einfacher Art und Weise ermöglicht, Patientenmessdaten von einer Außenstation 3 in üblicher Art und Weise an einen anderen Ort, d.h. insbesondere einer Basisstation 2, zu ermitteln und diese erkennbar für einen Arzt in Echtzeit darzustellen.
Diese technische Lösung ermöglicht eine einfache, sichere und robuste Kommunikationsverbindung, beispielsweise eine Breitbandverbindung für eine AV-Verbindung (Audio-Video) zwischen zumindest einer Basisstation 2, einer Außenstation 3 und zumindest einer Zusatzstation 4.

Die Zusatzstation 4 ist ein externer interdisziplinärer Telekonsultationspunkt, welcher insbesondere die Einbindung der folgenden Fachbereiche:
Hals-, Nasen-, Ohrenheilkunde,
Gastroenterologie (zum Beispiel Rektoskopie),
Gynäkologie (äußere),
Dermatologie,
Kardiologie,
Orthopädie, Psychiatrie, Neurologie,
Urologie (äußere) und
Chirurgie
im Rahmen einer Telefonkonferenz und Beiziehung von weiterer spezieller medizinischer Fachkompetenz, wie Apotheke oder anderer Dienstleister im medizinischen Bereich, ermöglicht.

Übertragungseinrichtung zur drahtlosen Übermittlung von Patientendaten, mit zumindest mit einer Basisstation angeordnet am Praxisstandort und zumindest einer Außenstation angeordnet am Patientenstandort, wobei die Außenstation zumindest eine Datenschnittstelle zur Übernahme von Patientendaten, welche durch Messungen von zumindest einem Messgerät am lebenden Messobjekt ermittelt worden, besitzt. Die Datenübermittlung erfolgt via aller heute diesbezüglich üblicher Medien, insbesondere via Mail und/oder als livestream.

Zweckmäßig ist diesbezüglich eine vor Ort, nämlich an der Außenstation, von uns ausgebildete Nicht-ärztliche Praxisassistenz (NäPA) oder erfahrene Schwester, die mit einem entsprechenden Tablett zu bestimmten Zeiten Sprechstunden abhält (Patient kommt zur Videosprechstunde), Hausbesuche fährt oder Pflegeheime kontaktiert.
In unserem Modell wird die reine Videosprechstunde, also der Blickkontakt über die NäPA hergestellt zwischen Patienten und Arzt, ergänzt durch Diagnostik der kameraerfassbaren körperlichen Oberflächen des Patienten, realisiert.

Diese Lösung kann eine Kameratechnik-Einheit (Telepack X) mit einer Mikroprozessoreinheit, beispielsweise einem PC, und einer Softwarelösung zum Übertragen der Bilder und Filme in Echtzeit, eine Telefonanbindung und verschiedene technische Module umfassen.
Damit lassen sich insbesondere Blutdruck, Blutzucker, Blutgas, EKG, die Sondierung des Rachen-, des Mundes, des Ohr-, des Nasenraumes, die Inspektion von Haut und Wunden, des äußeren Genitales und des Afters, Gangbild, Bewegungsabläufe und Sauerstoffsättigung teleportieren.
In den Bilddaten werden regelmäßig keine Patienteninformationen gesendet.
Für diese Messungen ist es erforderlich, dass das jeweilige Messgerät mit dem zu messenden Subjekt verbunden wird und/ oder dass die jeweiligen Messgrößen in geeigneter, bekannter Art und Weise gemessen oder erfasst und häufig auch verarbeitet und weitergeleitet werden.

Ob eine Messung durch Kontakt mit dem Patienten oder kontaktlos erfolgen kann, ist abhängig von der Körpereigenschaft, der Körperfunktion bzw. dem Vitalparameter der gemessen werden soll und der dazu verwendeten Messmethode.
Die am Teleconsulting teilnehmenden Ärzte, insbesondere als Bedienperson 5 und 7 bezeichnet, können sich über die Videokonferenz oder per Telefon über den Patienten austauschen.

Videosprechstunden per Videokonferenz können beim diesbezüglichen Patientenkontakt technisch relativ problemlos realisieren werden. Diese dienen vorranging dazu, chronische Erkrankungen zu begleiten und bei Akuterkrankungen zu entscheiden, ob tatsächlich ein direkter Arzt-Patientenkontakt erforderlich oder ein Krankenhaus aufzusuchen ist oder ob die vor Ort vorhandene nicht-ärztliche Praxisassistents (NäPA), Schwester, Pflegerin oder der telemedizierende Hausarzt in Ruhe eine Behandlung vornehmen kann oder der Patient einen Facharzt aufsuchen muss.

Dies lässt sich durch den Einsatz einer suffizienten Telemedizin, die haus- und fachärztliches Fachwissen (know how) in Echtzeit durch livestream- Audio-Videokonferenzen und direkter Befunderhebung abmildern.

Die Telemedizin ersetzt nicht auf Dauer und prinzipiell den persönlichen Arzt-PatientenKontakt. Es sei denn in einem Gebiet ohne medizinische Versorgung. Dann könnte die Versorgung, wie im Wesentlichen, bis auf Notfälle und schwere Erkrankungen, ausschließlich und suffizient über diese Art von Betreuung erfolgen.
Des Weiteren lassen sich über eine komfortable Telemedizineinheit Pflegeheime aus der Ferne auch fachärztlich medizinisch betreuen.

Außerdem ist es ermöglicht, eine Telefonkonferenz zwischen Arzt und Patienten und/oder der Bedienperson 7, insbesondere einer medizinisch ausgebildeten Person, insbesondere einem Facharzt, zu schalten und durchzuführen.

Ein Konferenz-Endgerät kann im Sinne der Erfindung ein üblicher PC, Laptop, Tablett oder Mobiltelefon (Smartphone) sein, welches über eine übliche Software für die Durchführung einer Audio-Videokonferenz und Messwertanzeige verfügen.

Die abhängigen Ansprüche 2 bis 5 enthalten vorteilhafte Ausgestaltungen der Erfindung ohne diese damit zu begrenzen.

Bevorzugt ist, dass zumindest eine Zusatzstation 4 mit der Basisstation 2 in Echtzeit verbindbar ist, wobei der Bedienperson 7 der Zusatzstation 4 die Patientendaten visuell und/oder akustisch zur Verfügung stehen.
Facharztrichtungen der Bedienperson 7 sollten insbesondere sein: Chirurg, Urologe, Neurologe, Gynäkologe, HNO, Dermatologe, Physiologe, Orthopäde, Psychologe und/oder Kardiologie.

Damit ist es möglich, insbesondere visuelle, endoskopische, dermatoskopische Echtzeit-Video-Befunde auf Abruf den angeschlossenen Fachärzten zu präsentieren, die Diagnose gemeinsam zu stellen und die Therapie festzulegen.
Bei der visuellen Befundung werden oberflächenerfassbare und abbildbare Körperoberflächen einbezogen.

Dabei kann es sich um eine Facharzt-zu-Facharzt-Verbindung, Hausarzt-zu-Facharzt-Verbindung, Facharzt-zu-Hausarzt-Verbindung oder Pflegeheim-zu-Arzt-Verbindung handeln.
Weiterhin ist es denkbar, Verbindungen zwischen Dienstleistungsunternehmen (Apotheken, Hörgeräteakustiker, Psychotherapeuten, Psychologen, Ergotherapeuten) und Fachärzten zu ermöglichen.

Bevorzugt ist, dass die Außenstation 3 eine von einer Person, insbesondere der Bedienperson 6, tragbare Station, mit einem maximalen Gewicht von 10 kg, ist.
Die Außenstation 3 wird möglichst technisch einfach, bautechnisch klein und auch von einer weiblichen Person gut zu transportieren sein, um den diesbezüglichen Transportaufwand möglichst gering zu halten.
Die Art und Weise der Auswahl der technischen Bauteile der Außenstation 3 ist diesbezüglich abgestimmt.
Solche technischen Bauteile der Außenstation 3 sind beispielsweise ein Laptop, ein Handy, ein Handyaufsatz Endoskopie, ein Handyaufsatz Dermatoskop sowie ein Endoskop. Erforderlich ist ein Internetzugang mit mindestens ADSL 6000 Übertragungsqualität. Die Lösung erfüllt die Grundsätze des aktuellen Standesrechts und allgemeiner Rechtsanforderungen (z.B. Datensicherheit).

Bevorzugt ist, dass die Außenstation 3 zumindest ein EKG, insbesondere ein Smartphone- / Smartwatch-EKG, ein Stethoskop, ein Dermatoskop, ein Endoskop, eine Digitalkamera und ein Pulsoximeter umfasst, welche jeweils eine Datenschnittstelle zur drahtgebundenen und/oder drahtlosen Übertragung von gemessenen Vitaldaten über die Datenschnittstelle zur Außenstation 3 besitzen.

Im Ergebnis umfangreicher Tests wurde die vorstehende Auswahl der vorgenannten elektronischen Geräte getroffen. Dabei war wesentlich, dass nur solche Geräte zur Außenstation gehören, welche statistisch oft zum Befunden benötigt werden und insgesamt durch eine Bedienperson 6, oft eine weibliche Person, zum Patientenstandort getragen werden können. Das Gesamtgewicht der Außenstation beträgt somit maximal 10 kg.
Die vorgenannten Komponenten der Außenstation können in einem üblichen Transportbehältnis, wie einem Rucksack, Koffer oder dgl., verstaut sein.

Eine Digitalkamera ist im Sinne der Erfindung eine Kamera, die als Aufnahmemedium anstatt eines Films ein digitales Speichermedium verwendet; das Bild wird zuvor mittels eines elektronischen Bildwandlers digitalisiert. Digitalkameras sind in andere Geräte integriert. Alle modernen Mobiltelefone und Smartphones enthalten eine eingebaute Digitalkamera.

Bevorzugt ist, dass eine Breitbandverbindung für zumindest eine Audio-Video -Verbindung zwischen zumindest der Basisstation 2 und der Zusatzstation 4 besteht.
Im Sinne der Erfindung ist eine Breitbandverbindung für zumindest eine Audio-Video - Verbindung eine solche, wie zumindest derzeit in Deutschland üblich und bekannt.

Bevorzugt ist, dass die Basisstation 2, die Außenstation 3 und die Zusatzstation 4 zumindest jeweils eine Sende-, Empfangs-, Anzeige- und/oder Wiedergabevorrichtung für visuelle und/oder klangliche Signale und/oder Patientenmessdaten besitzt.

Die Aufgabe der Erfindung wird durch ein Verfahren mit den Merkmalen gemäß Anspruch 6 gelöst.

Erfindungswesentlich ist dabei, dass das Verfahren zur Bereitstellung von Patientenmessdaten von lebenden Messobjekten an zumindest einer Basisstation 2 in Echtzeit, wobei zumindest eine Kommunikationsverbindung, insbesondere eine A/V- Verbindung, zwischen der Basisstation 2 und der Außenstation 3 durch eine Bedienperson 6 hergestellt wird, durch die Bedienperson 6 die Übermittlung von Patientendaten an die Basisstation 2 in Echtzeit eingeleitet wird, durch die Bedienperson 5 an der Basisstation 2 dabei ein Monitoring dieses Verfahrensschrittes und/oder Vorbereitung und Durchführung der Erhebung von Messdaten erfolgt, und durch die Bedienperson 5 eine Prüfung und/oder eine Auswertung der übermittelten Patientendaten (und Veranlassung weiterer Aktivitäten) erfolgt.
Es ist ermöglicht, dass während der gesamten Audio-Vidokonferenz ein Sichtkontakt zwischen dem Arzt und Patienten besteht.

Die abhängigen Ansprüche 7 und 8 enthalten vorteilhafte Ausgestaltungen der Erfindung ohne diese damit zu begrenzen.

Die Aufgabe der Erfindung wird durch eine Verwendung der erfindungsgemäßen Übertragungseinrichtung mit den Merkmalen gemäß Anspruch 9 gelöst.

Eine mögliche Anwendung könnte das Vorstellen unklarer oder komplizierter Befunde und das Einholen einer Zweitmeinung sein.

Eine persönliche Vorstellung des Patienten ist davon unabhängig jederzeit möglich.

Um einen Sofortkontakt einer Videokonferenz zu sichern, sollten je Fachrichtung zumindest mehrere Fachärzte beteiligt und auswählbar sein. Außerdem ist ermöglicht, einen Facharzt auch nachträglich einzubeziehen.

### Beispiel: 1

### Ablaufbeschreibung eines Telekonsils anhand einer HNO-Erkrankung

Eine Schwester, nämlich eine Bedienperson 6, fährt mit einem PKW, Moped oder Fahrrad zu einem Patienten an den Patientenstandort, welcher beispielsweise ein Pflegeheim oder Wohnung des Patienten sein kann.
Diese Schwester nimmt im livestream Kontakt zum Hausarzt bzw. einer Bedienperson 5 auf, welcher sich an der Basisstation 2 am Praxisstandort befindet. Damit ist der Kontakt zwischen dem Patienten, der Schwester und Arzt im livestream hergestellt. Dabei kann ein Blickkontakt zwischen Arzt und Patienten hergestellt werden, wobei beispielsweise eine integrierte Digitalkamera eines iPads oder iPhones, positioniert auf einem Stativ, in üblicher Art und Weise genutzt wird.
Nachfolgend erfolgt die Durchführung von Untersuchungen am Patienten auf Weisung des zugeschalteten Arztes.
Die Erhebung von Befunden erfolgt mithin in Echtzeit, beispielsweise EKG usw. wird dem Arzt in Echtzeit zugänglich.
Der Arzt kann ggf. Instrumente - unter Blickkontakt - durch entsprechende Anweisungen an die Schwester steuern, z.B. ein Endoskop/Kamera, über die Schwester, führt somit virtuell deren Hand, schaut in Rachen oder Ohr, d.h. Arzt schaut nicht nur übermittelte Bilder an, sondern den Befund, zum Beispiel Hautfleck direkt und steuert die Tele-Technik über die Schwester, die damit der verlängerte Arm des Arztes ist, sie ist quasi seine direkte Assistenz.
Der Arzt ist also "vor Ort" dabei, Schaltung im livestream additiv zu anderen Ärzten ist optional, sprich bei Bedarf möglich und schaltet also direkt andere Fachkompetenz zu, quasi Dreiecksvideokonferenz: Arzt-Patient/Schwester-Facharzt.

Dabei erfolgt eine Bereitstellung von Patientendaten an zumindest dieser einen Basisstation 2 in Echtzeit, wobei zumindest eine Kommunikationsverbindung, insbesondere eine A/V-Verbindung, zwischen der Basisstation 2 und der Außenstation 3 durch eine Bedienperson 6 hergestellt wird.
Der Bedienperson 5 an der Basisstation 2 ist ein Monitoring dieser Verfahrensschritte und/oder Vorbereitung und Durchführung der Erhebung von Patientenmessdaten ermöglicht. Somit kann durch den Hausarzt, die Bedienperson 5, eine Prüfung und eine Auswertung der übermittelten Patientendaten in Echtzeit erfolgen und im Bedarfsfall einen Facharzt in Echtzeit beiziehen.

Die aktuell ansprechbaren Fachärzte können beispielsweise in einer computergestützten Datenbank ersichtlich sein, so dass diese in Echtzeit zugeschaltet werden können.

Dazu wird eine Kommunikationsverbindung zu einem externen Facharzt hergestellt, wobei dabei zumindest eine Zusatzstation 4 mit der Basisstation 2 in Echtzeit verbunden ist. Der Bedienperson 7, d.h. dem Facharzt, werden über die Zusatzstation 4 zumindest ein Teil der Patientendaten visuell und/oder akustisch zur Verfügung gestellt, welche an der Basisstation 2 anliegen.

Also besteht damit ein "direktes" Sprechzimmer, wo der Patient dem Arzt "gegenüber" sitzt, die Schwester körperlich anwesend ist und Anweisungen direkt ausführt. Außerdem kommt der Facharzt virtuell ins Zimmer und es wird ein Konsil im Beisein des Patienten durchgeführt.
1) Datenübertragung von Vitaldaten zum Arzt: Temperatur, Blutdruck, Blutzucker, Pulsoxymeter, Gewicht (Waage), Spirometer, EKG usw.
2) Übertragung von Fotos: Dematoskopie/Kamera
3) Übertragung von (live) Videos: Endoskope/Kamera

### Beispiel: A

### Technische Voraussetzungen:

Breitbandverbindung für eine AV-Verbindung (Audio-Video) zwischen zwei und mehr Praxisstandorten.

### Beispiel: B

### Technische Voraussetzungen:

- Praxis PC, Headset
- Bereitstellung eines 6000 ADSL Anschlusses
- Datensafe (Server)
- Endoskopieeinheit: Telepack
- Endoskop (HNO)
- Dermatoskop (Derma)
- Rektoskop (Uro)
- zweite Kamera zuschaltbar (z.B. für Tele-Endoskopie)

### Beispiel: 2

### Ablaufbeschreibung Telekonsil anhand einer HNO-Erkrankung

Ein Patient geht zum Allgemeinarzt/Hausarzt wegen HNO-Beschwerden. Der Arzt tritt über das Teleconsulting mit dem Facharzt in Verbindung, untersucht den Patienten mit dem Endoskop mithilfe des Facharztes und bespricht die Diagnose mit dem HNO-Arzt. Die Digitalkamera des Mobiltelefons (Smartphones) oder Tabletts wird auf Ständer angeordnet und auf den Patienten gerichtet, so dass Arzt den Patienten sieht. Die Schwester nimmt eine Messung mit Hilfe eines der Messgeräte der Außenstation in üblicher Art und Weise vor. Die gewonnenen Patientenmessdaten werden gesondert, d.h. teils über die Digitalkamera des Mobiltelefons (Smartphones) oder Tabletts, zum Hausarzt und/oder Facharzt in Echtzeit übertragen.
Der Allgemeinarzt veranlasst alle weiteren notwendigen Befunderhebungen. Der Patient geht, wenn dann noch erforderlich, mit komplett erhobenen Befunden zum HNO-Arzt für die Therapie.
Für den Patienten ergeben sich daraus eine Vielzahl von Vorteilen: Er bekommt fachärztliche Hilfe bereits beim ersten Arztbesuch, er muss weniger Wege- und Wartezeiten beim Allgemein- und Facharzt in Kauf nehmen sowie eine Verkürzung des gesamten Behandlungsprozesses ist möglich.

## Patentansprüche

1. Audio-Videokonferenzsystem, welches jeweils ein Konferenz-Endgerät an einer Basisstation (2), welche an einem Praxisstandort geordnet ist, und einer Außenstation (3), welche an einem Patientenstandort angeordnet ist, besitzt, wobei zumindest eine Zusatzstation (4) zur Basisstation (2) zuschaltbar ist, **dadurch gekennzeichnet, dass** das Audio-Videokonferenzsystem eine Übertragungseinrichtung (1) zur drahtlosen Übermittlung von Patientenmessdaten in Echtzeit von der Außenstation (3) an die Basisstation (2) und/oder Zusatzstation (4) besitzt, wobei die Außenstation (3) zumindest eine Datenschnittstelle zur Übernahme von Patientenmessdaten, welche durch Messungen von zumindest einem medizinischen Messgerät (8) am Patienten (9) ermittelt worden sind, besitzt und die Außenstation (3), welche zumindest ein medizinisches Messgerät (8) umfasst, tragbar ist.

2. Audio-Videokonferenzsystem gemäß Anspruch 1, **dadurch gekennzeichnet, dass** zumindest eine Zusatzstation (4) mit der Basisstation (2) in Echtzeit verbindbar ist, wobei der Bedienperson (7) der Zusatzstation (4) die Patientendaten visuell und/oder akustisch zur Verfügung stehen.

3. Audio-Videokonferenzsystem gemäß Anspruch 1, **dadurch gekennzeichnet, dass** die Außenstation (3) eine von einer Person, insbesondere der Bedienperson (6), tragbare Station, mit einem maximalen Gewicht von 10 kg, ist.

4. Audio-Videokonferenzsystem gemäß Anspruch 1, **dadurch gekennzeichnet, dass** die Basisstation (2), die Außenstation (3) und die Zusatzstation (4) zumindest jeweils eine Sende-, Empfangs-, Anzeige- und/oder Wiedergabevorrichtung für visuelle und/oder klangliche Signale und/oder Messdaten besitzen.

5. Audio-Videokonferenzsystem gemäß Anspruch 1, **dadurch gekennzeichnet, dass** die Außenstation (3) zumindest ein EKG, insbesondere ein Smartphone-/ Smartwatch-EKG, ein Stethoskop, ein Dermatoskop, ein Endoskop, eine Digitalkamera und ein Pulsoximeter umfasst, welche jeweils eine Datenschnittstelle zur drahtgebundenen und/oder drahtlosen Übertragung von gemessenen Vitaldaten über die Datenschnittstelle zur Außenstation (3) besitzen.

6. Verfahren zur Durchführung einer Audio-Videokonferenz und zusätzlich dazu zur Bereitstellung von Patientenmessdaten an zumindest einer Basisstation (2) in Echtzeit, wobei zumindest eine Kommunikationsverbindung zwischen der Basisstation (2) und der Außenstation (3) durch eine Bedienperson (6) hergestellt wird, durch die Bedienperson (6) die Erhebung und Übermittlung von Patientenmessdaten an die Basisstation (2) in Echtzeit eingeleitet wird, durch die Bedienperson (5) an der Basisstation (2) dabei ein Monitoring dieses Verfahrensschrittes und/oder Vorbereitung und Durchführung der Erhebung von Messdaten erfolgt, durch die Bedienperson (5) eine Prüfung und eine Auswertung der übermittelten Patientendaten erfolgt.

7. Verfahren gemäß Anspruch 6, **dadurch gekennzeichnet, dass** zur Prüfung der Patientenmessdaten eine Verbindung von der Basisstation (2) zu zumindest einer Zusatzstation (4) in Echtzeit hergestellt wird und die Bedienperson (7) der Zusatzstation (4) diese Patientenmessdaten in Echtzeit befundet.

8. Verfahren gemäß Anspruch 6 oder Anspruch 7, **dadurch gekennzeichnet, dass** eine Erhebung von Befunden in Echtzeit durch die Bedienperson (5) und/oder die Bedienperson (7) erfolgt.

9. Verwendung des Audio-Videokonferenzsystems gemäß einem der Ansprüche 1 bis 5 für Audio-Videokonferenzen und Messdatenübertragung in Echtzeit in der Telemedizin.
